(19) Europäisches Patentamt
European Patent Office
Office européen des brevets

(11) Publication number: **0 276 500 B1**

(12) **EUROPEAN PATENT SPECIFICATION**

(45) Date of publication of patent specification: **15.05.91**

(51) Int. Cl.5: **C07D 209/34**, C07D 405/06, C07D 409/06

(21) Application number: **87201673.8**

(60) Publication number of the earlier application in accordance with Art.76 EPC: **0 153 818**

(22) Date of filing: **04.02.85**

(54) **2-Oxindole intermediates.**

(30) Priority: **07.02.84 US 577903**
**12.06.84 US 619861**
**13.11.84 US 670697**

(43) Date of publication of application:
**03.08.88 Bulletin 88/31**

(45) Publication of the grant of the patent:
**15.05.91 Bulletin 91/20**

(84) Designated Contracting States:
**AT BE CH DE FR GB IT LI LU NL SE**

(56) References cited:
**EP-A- 0 155 828**
**EP-A- 0 156 603**
**EP-A- 0 164 860**
**EP-A- 0 175 551**

**CHEMICAL ABSTRACTS, vol. 43, no. 4, 25th February 1949, column 1411d-1412f, Columbus, Ohio, US; N.N. MAXIM et al.: "Action of cyclohexyl methyl ketone on isatin. Preparation of cyclohexylcinchoninic acid"**

(73) Proprietor: **PFIZER INC.**
**235 East 42nd Street**
**New York, N.Y. 10017(US)**

(72) Inventor: **Kadin, Saul Bernard**
**10, Eldane Street**
**New London New London County Connecticut(US)**

(74) Representative: **Wood, David John et al**
**PFIZER LIMITED, Ramsgate Road**
**Sandwich, Kent CT13 9NJ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 276 500 B1

## Description

This application is a divisional of EP-A-0153818. It concerns synthetic intermediates of the formula:

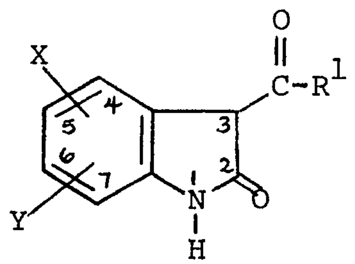

--- (A)

and the base salts thereof,
wherein X is hydrogen, 5-fluoro or 5-chloro; Y is hydrogen, 6-fluoro or 6-chloro; and $R^1$ is benzyl, furyl, thienyl or thienylmethyl; provided that when X and Y are both hydrogen, $R^1$ is not benzyl.

Preferred compounds of the formula (A) are those wherein (a) X is 5-chloro, Y is hydrogen and $R^1$ is 2-thienyl and (b) X is 5-fluoro, Y is 6-chloro and $R^1$ is 2-thienyl.

The compounds of the formula (A) are synthetic intermediates useful in the preparation of the N-substituted oxindole-l-carboxamide antiinflammatory agents of the parent application EP-A-0153818 as is described in that application.

The compounds of formula (A) are prepared from the appropriate 2-oxindole compound of the formula:

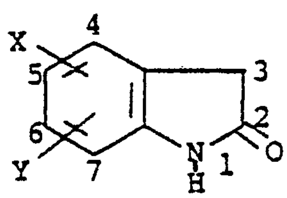

--- (B)

wherein X and Y are as defined previously. This is accomplished by attaching the substituent $-C(=O)-R^1$ to the 3-position.

The $-C(=O)-R^1$ side-chain can be attached to a compound of the formula (B) by reaction with a derivative of the appropriate acid of the formula $R^1-C(=O)-OH$, in a lower-alkanol solvent (e.g. ethanol), in the presence of an alkali metal salt of the lower-alkanol solvent (e.g. sodium ethoxide), according to standard procedures. Typical derivatives of the acid of the formula $R^1-C(=O)OH$ which can be used include acid chlorides, acid anhydrides of the formula $R^1-C(=O)-O-C(=O)-R^1$, $R^1-C(=O)-O-C(=O)-R^2$ and $R^1-C(=O)-O-C(=O)-OR^3$, and simple alkyl esters of the formula $R^1-C(=O)-OR^3$, wherein $R^2$ is a bulky lower alkyl group such as t-butyl, and $R^3$ is a lower alkyl group. Usually, a small excess of the derivative of the acid of formula $R^1-C(=O)-OH$ is used, and the alkoxide salt is usually present in an amount from one to two molar equivalents, based on said derivative of the acid of formula $R^1-C(=O)OH$. The reaction between the derivative of the acid of the formula $R^1-C(=O)OH$ and the compound of formula (B) is usually started at 0 to 25 °C., but it is then usual to heat the reaction mixture at a temperature in the range from 50 to 130 °C., and preferably at about 80 °C., to complete the reaction. Under these circumstances, reaction times of a few hours, e.g. two hours, up to a few days, e.g., two days, are commonly used. The reaction mixture is then cooled, diluted with an excess of water, and acidified. The product of formula (A) can then be recovered by filtration or by the standard procedure of solvent extraction.

The 2-oxindole compounds of formula (B) are prepared by known methods, or methods analogues to know methods. Consult: "Rodd's Chemistry of Carbon Compounds," Second Edition, S. Coffey editor, Volume IV Part A, Elsevier Scientific Publishing Company, 1973, pp. 448-450; Gassman et al., Journal of Organic Chemistry, 42, 1340 (1977); Wright et al., Journal of the American Chemical Society, 78, 221 (1956); Beckett et al., Tetrahedron, 24, 6093 (1968) ; United States Patents Nos. 3,882,236, 4,006,161 and 4,160,032; Walker, Journal of the American Chemical Society, 77, 3844 (1955) ; Protiva et al., Collection of Czechoslovakian Chemical Communications, 44, 2108 (1979); McEvoy et al., Journal of Organic Chemistry, 38, 3350

(1973); Simet, Journal of Organic Chemistry, 28, 3580 (1963); Wieland et al., Chemische Berichte, 96, 253 (1963); and references cited therein.

The following Examples illustrate the invention:-

EXAMPLE 1

3-(2-Furoyl)-2-oxindole

To a stirred solution of 5.5 g (0.24 mole) of sodium in 150 ml of ethanol was added 13.3 g (0.10 mole) of 2-oxindole at room temperature. The resulting slurry was cooled to ice-bath temperature, and then 15.7 g (0.12 mole) of 2-furoyl chloride was added, dropwise, during 10-15 minutes. The ice-bath was removed, and additional 100 ml of ethanol was added and then the reaction mixture was heated under reflux for 7 hours. The reaction mixture was allowed to stand overnight and then the solid was filtered off. The solid was added to 400 ml of water and the resulting mixture was acidified using concentrated hydrochloric acid. The mixture was cooled with ice and the solid was collected by filtration. The solid residue was recrystallized from 150 ml of acetic acid, affording 8.3 g of yellow crystals, mp 209-210 (dec).

Analysis: Calcd. for $C_{13}H_9O_3N$: C, 68.72; H, 3.99; N, 6.17%. Found: C, 68.25; H, 4.05; N, 6.20%.

EXAMPLE 2

Reaction of 2-oxindole with the appropriate acid chloride using the method of Example I, gave the following additional products:

3-(2-thenoyl)-2-oxindole, mp 189-190 °C, 17% yield;

3-(2-[2-thienyl]acetyl)-2-oxindole, mp 191-192.5 °C, 38% yield;

5-Chloro-3-(2-[2-thienyl]acetyl)-2-oxindole, mp 228-230 °C., 22% yield.

EXAMPLE 3

3-(3-Furoyl)-2-oxindole

To a stirred solution of 2.8 g (0.12 mole) of sodium in 200 ml of ethanol was added 13.3 g (0.10 mole) of 2-oxindole, followed by 16.8 g of ethyl 3-furoate. The mixture was heated under reflux for 47 hours, cooled and then the solvent was removed by evaporation in vacuo. The residue was triturated under 200 ml of ether, and the solid was collected by filtration and discarded. The filtrate was evaporated in vacuo, and the residue triturated under diisopropyl ether and recovered by filtration. The solid was suspended in 250 ml of water, which was then acidified with concentrated hydrochloric acid. This mixture was stirred to give a solid, which was recovered by filtration. This latter solid was recrystallized from acetic acid followed by acetonitrile to give 705 mg of the title compound, mp 185-186 °C.

Analysis: Calcd. for $C_{13}H_9O_3N$: C, 68.72; H, 3.99; N, 6.17%. Found: C, 68.72; H, 4.14; N, 6.14%.

EXAMPLE 4

Reaction of the appropriate 2-oxindole with the ethyl ester of the requisite carboxylic acid, substantially according to the procedure of Example 3, gave the following compounds:

5-chloro-3-(2-thenoyl)-2-oxindole, mp 190.5-192 °C., 36% yield;

5-chloro-3-(2-furoyl)-2-oxindole, mp 234-235 °C., 54% yield;

5-chloro-3-(2-phenylacetyl)-2-oxindole, mp 241-243 °C., 61% yield;

5-fluoro-3-(2-furoyl)-2-oxindole, mp. 222-224 °C., 51% yield;

5-fluoro-3-(2-thenoyl)-2-oxindole, mp 200-203 °C., 26% yield;

6-fluoro-3-(2-furoyl)-2-oxindole, mp 239-242 °C., 26% yield; and

6-chloro-5-fluoro-3-(2-thenoyl)-2-oxindole, mp 212-215 °C., 20% yield.

The following Preparations describe the preparation of certain starting materials used in the previous Examples:-

PREPARATION 1

5-Chloro-2-oxindole

To a stirred slurry of 100 g (0.55 mol) of 5-chloroisatin in 930 ml of ethanol was added 40 ml (0.826 mol) of hydrazine hydrate, resulting in a red solution. The solution was heated under reflux for 3.5 hours, during which time a precipitate appeared. The reaction mixture was stirred overnight, and then the precipitate was recovered by filtration to give 5-chloro-3-hydrazono-2-oxindole as a yellow solid, which was dried in a vacuum oven. The dried solid weighed l05.4 g.

The dried solid was then added portionwise, during l0 minutes, to a solution of l25.l g of sodium methoxide in 900 ml of absolute ethanol. The resultant solution was heated under reflux for 10 minutes and then it was concentrated in vacuo to a gummy solid. The gummy solid was dissolved in 400 ml of water and the aqueous solution thus obtained was decolorized with activated carbon and then poured into a mixture of l liter of water and l80 ml of concentrated hydrochloric acid containing ice chips. A tan solid precipitated and it was collected by filtration and washed thoroughly with water. The solid was dried and then it was washed with diethyl ether. Finally it was recrystallized from ethanol to give 48.9 g of the title compound, mp l93-l95 ° C. (dec).

PREPARATION 2

5-Fluoro-2-oxindole

To a stirred solution of 11.1 g (0.1 mol) of 4-fluoroaniline in 200 ml of dichloromethane, at -60 to -65 ° C, was added, dropwise, a solution of 10.8 g (0.1 mol) of t-butyl hypochlorite in 25 ml of dichloromethane. Stirring was continued for 10 minutes at -60 to -65 ° C, and then was added, dropwise, a solution of l3.4 g (0.l mol) of ethyl 2-(methylthio)acetate in 25 ml of dichloromethane. Stirring was continued at -60 ° C. for l hour and then was added, dropwise, at -60 to -65 ° C, a solution of 11.1 g (0.11 mol) of triethylamine in 25 ml of dichloromethane. The cooling bath was removed, and when the reaction mixture had warmed to room temperature, l00 ml of water was added. The phases were separated, and the organic phase was washed with saturated sodium chloride solution, dried ($Na_2SO_4$) and evaporated in vacuo. The residue was dissolved in 350 ml of diethyl ether, to which was added 40 ml of 2N hydrochloric acid. This mixture was stirred at room temperature overnight. The phases were separated and the ether phase was washed with water, followed saturated sodium chloride. The dried ($Na_2SO_4$) ether phase was evaporated in vacuo to give l7 g of an orange-brown solid which was triturated under isopropyl ether. The solid was then recrystallized form ethanol, to give 5.58 g of 5-fluoro-3-methylthio-2-oxindole, mp l5l.5-l52.5 ° C.

Analysis: Calcd for $C_9H_8ONFS$: C, 54.80; H, 4.09; N, 7.l0%. Found: C, 54.74; H, 4.ll; N, 7.ll%.

A sample of the above 5-fluoro-3-methylthio-2-oxindole (986 mg, 5.0 mmol) was added to 2 teaspoonsful of Raney nickel under 50 ml of absolute ethanol, and then the reaction mixture was heated under reflux for 2 hours. The catalyst was removed by decantation and was washed with absolute ethanol. The combined ethanol solutions were evaporated in vacuo and the residue was dissolved in dichloromethane. The dichloromethane solution was dried ($Na_2SO_4$) and evaporated in vacuo to give 475 mg of 5-fluoro-2-oxindole, mp l2l-l34 ° C.

PREPARATION 3

6-Chloro-5-fluoro-2-oxindole

To l30 ml of toluene was added, with stirring, 24.0 g (0.l65 mole) of 3-chloro-4-fluoroaniline and l3.5 ml (0.l66 mole) of pyridine. The resulting solution was cooled to ca 0 ° C. and l3.2 ml (0.l66 mole) of 2-chloroacetyl chloride was added. The reaction mixture was stirred at room temperature for 5 hours and then it was extracted twice with l00 ml of lN hydrochloric acid, followed by l00 ml of saturated sodium chloride solution. The resulting toluene solution was dried using magnesium sulfate, and then it was concentrated in vacuo to give 32.6 g (88% yield) of N-(2-chlorcacetyl)-3-chloro-4-fluoroaniline.

A 26.63-g sample of the N-(2-chloroacetyl) -chlcro-4-fluoroaniline was thoroughly mixed with 64 g of anhydrous aluminum chloride, and the mixture was heated at 2l0-230 ° C. for 8.5 hours. The reaction mixture was then poured onto a mixture of ice and lN hydrochloric acid, with stirring. Stirring was continued for 30 minutes, and then the solid was collected by filtration (22.0 g). The solid was dissolved in l:l ethyl acetate-hexane and chromatographed on 800 g of silica gel. Elution of the column, followed by evaporation of the fractions, produced ll.7 g of the N-(2-chloroacetyl)-3-chloro-4fluorcaniline, followed by 3.0 g of 6-chloro-5-fluoro-2-oxindole. The latter material was recrystallized from toluene to give l.70 g (7% yield) of the title

compound, mp l96-206 °C. Analysis by NMR spectroscopy indicated that the product was contaminated by some 4-chloro-5-fluoro-2-oxindole. A second crop weighing 0.8 g was obtained.

## Claims

1. A compound of the formula:-

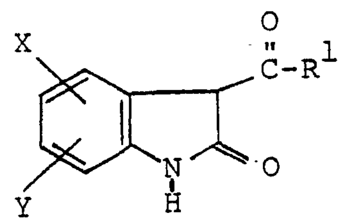

--- (A)

and the base salts thereof, wherein X is hydrogen, 5-fluoro or 5-chloro; Y is hydrogen, 6-fluoro or 6-chloro; and $R^1$ is benzyl, furyl, thienyl or thienylmethyl; provided that when X and Y are both hydrogen, $R^1$ is not benzyl.

2. The compound of claim 1 wherein X is 5-chloro, Y is hydrogen and $R^1$ is 2-thienyl.

3. The compound of claim 1 wherein X is 5-fluoro, Y is 6-chloro and $R^1$ is 2-thienyl.

Claims for the following Contracting State: AT

1. A process for preparing a compound of the formula:-

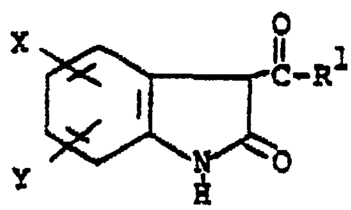

--- (A)

or a base salt thereof, wherein X is hydrogen, 5-fluoro or 5-chloro; Y is hydrogen, 6-fluoro or 6-chloro; and $R^1$ is benzyl, furyl, thienyl or thienylmethyl; provided that when X and Y are both hydrogen, $R^1$ is not benzyl;
<u>characterised in that</u> a compound of the formula:-

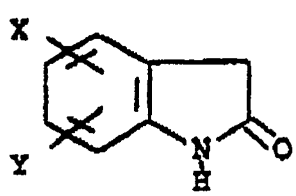

--- (B)

is reacted with an activated derivative of an acid of the formula $R^1$-C(=O)-OH, X, Y and $R^1$ being as defined for formula (A).

2. The process of claim l wherein X is 5-chloro, Y is hydrogen and $R^1$ is 2-thienyl.

3. The process of claim I wherein X is 5-fluoro, Y is 6-chloro and $R^1$ is 2-thienyl.

4. A process according to any one of the preceding claims, wherein the activated derivative is an acid chloride of the acid, an acid anhydride of the formula

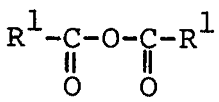, 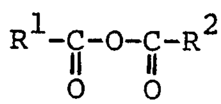 or 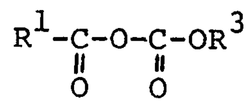

where $R^2$ is a bulky lower alkyl group and $R^3$ is a lower alkyl group, or an alkyl ester of the formula

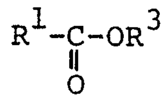

where $R^3$ is as defined above.

5. A process according to claim 4, wherein said bulky lower alkyl group is a t-butyl group.

6. A process according to any one of the preceding claims, characterised in that it is carried out in a lower alkanol solvent in the presence of an alkali metal salt of the alkanol.

7. A process according to claim 6, characterised in that it is carried out in ethanol in the presence of sodium ethoxide.

8. A compound of the formula (A) as defined in claim 1.

**Revendications**

1. Composé de formule :

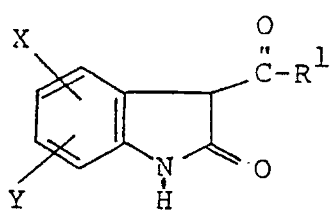

--- (A)

et ses sels de bases, formule dans laquelle X représente l'hydrogène, un radical 5-fluoro ou 5-chloro ; Y est l'hydrogène, un radical 6-fluoro ou 6-chloro ; et $R^1$ est un groupe benzyle, furyle, thiényle ou thiénylméthyle ; sous réserve que lorsque X et Y sont tous deux de l'hydrogène, $R^1$ ne soit pas un groupe benzyle.

2. Composé suivant la revendication 1, dans lequel X est un radical 5-chloro, Y est l'hydrogène et $R^1$ est un radical 2-thiényle.

3. Composé suivant la revendication 1, dans lequel X est un radical 5-fluoro, Y est un radical 6-chloro et $R^1$ est un radical 2-thiényle.

Revendications pour l'Etat contractant suivant : AT

1. Procédé de préparation d'un composé de formule :

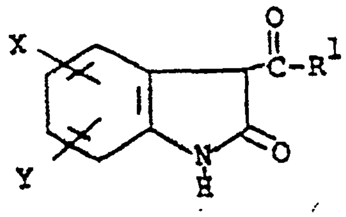

--- (A)

ou d'un sel de base de ce composé, formule dans laquelle X est l'hydrogène, un radical 5-fluoro ou 5-chloro ; Y est l'hydrogène, un radical 6-fluoro ou 6-chloro ; et $R^1$ est un radical benzyle, furyle, thiényle ou thiénylméthyle ; sous réserve que lorsque X et Y sont tous deux de l'hydrogène, $R^1$ ne soit pas un radical benzyle ;

caractérisé en ce qu'un composé de formule :

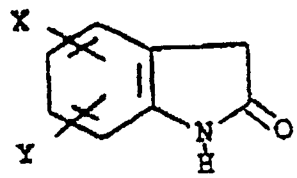

--- (B)

est amené à réagir avec un dérivé activé d'un acide de formule $R^1$-C(=O)-OH, X, Y et $R^1$ étant tels que définis pour la formule (A).

2. Procédé suivant la revendication 1, dans lequel X est un radical 5-chloro, Y est l'hydrogène et $R^1$ est un radical 2-thiényle.

3. Procédé suivant la revendication 1, dans lequel X est un radical 5-fluoro, Y est un radical 6-chloro et $R^1$ est un radical 2-thiényle.

4. Procédé suivant l'une quelconque des revendications précédentes, dans lequel le dérivé activé est un chlorure de l'acide, un anhydride d'acide de formule

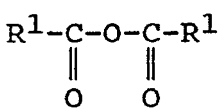, 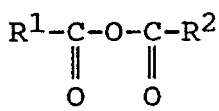 ou 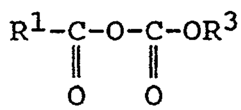

dans laquelle $R^2$ est un groupe alkyle inférieur volumineux et $R^3$ est un groupe alkyle inférieur, ou un ester d'alkyle de formule

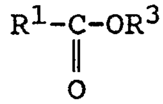

dans laquelle $R^3$ est tel que défini ci-dessus.

5. Procédé suivant la revendication 4, dans lequel le groupe alkyle inférieur volumineux est un groupe tertio-butyle.

6. Procédé suivant l'une quelconque des revendications précédentes, caractérisé en ce qu'il est mis en oeuvre dans un alcanol inférieur utilisé comme solvant en présence d'un sel de métal alcalin de l'alcanol.

7. Procédé suivant la revendication 6, caractérisé en ce qu'il est mis en oeuvre dans l'éthanol en présence d'éthylate de sodium.

8. Composé de formule (A) tel que défini dans la revendication 1.

**Ansprüche**

1. Verbindung der Formel

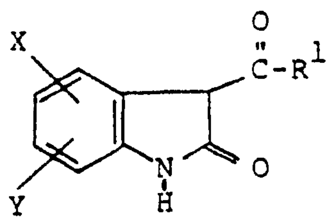

--- (A)

und deren Basensalze, worin X Wasserstoff, 5-Fluor oder 5-Chlor bedeutet; Y Wasserstoff, 6-Fluor oder 6-Chlor bedeutet und $R^1$ Benzyl, Furyl, Thienyl oder Thienylmethyl bedeutet, mit der Maßgabe, daß, wenn X und Y beide Wasserstoff bedeuten, $R^1$ nicht Benzyl ist.

2. Verbindung gemäß Anspruch 1, in welcher X 5-Chlor bedeutet, Y Wasserstoff bedeutet und $R^1$ 2-Thienyl bedeutet.

3. Verbindung gemäß Anspruch 1, in welcher X 5-Fluor bedeutet, Y 6-Chlor bedeutet und $R^1$ 2-Thienyl bedeutet.

Patentansprüche für folgenden Vertragsstaat : AT

1. Verfahren zur Herstellung einer Verbindung der Formel

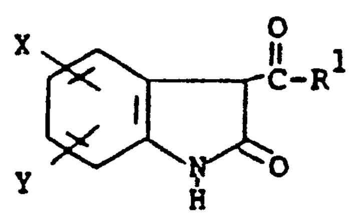

---(A)

oder eines Basensalzes derselben, worin X Wasserstoff, 5-Fluor oder 5-Chlor bedeutet; Y Wasserstoff, 6-Fluor oder 6-Chlor bedeutet und $R^1$ Benzyl, Furyl, Thienyl oder Thienylmethyl bedeutet, mit der Maßgabe, daß, wenn X und Y beide Wasserstoff bedeuten, $R^1$ nicht Benzyl ist, dadurch gekennzeichnet, daß eine Verbindung der Formel

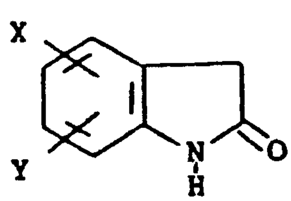

---(B)

mit einem aktivierten Derivat einer Säure der Formel $R^1$-C(=O)-OH umgesetzt wird, wobei X, Y und $R^1$ wie für Formel (A) definiert sind.

2. Verfahren gemäß Anspruch 1, in welchem X 5-Chlor bedeutet, Y Wasserstoff bedeutet und $R^1$ 2-Thienyl bedeutet.

3. Verfahren gemäß Anspruch 1, in welchem X 5-Fluor bedeutet, Y 6-Chlor bedeutet und $R^1$ 2-Thienyl bedeutet.

4. Verfahren gemäß irgendeinem der vorhergehenden Ansprüche, in welchem das aktivierte Derivat ein Säurechlorid der Säure, ein Säureanhydrid der Formel

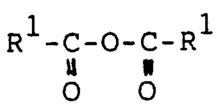 , 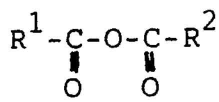 oder 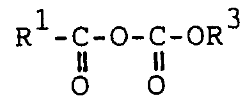

worin $R^2$ eine sperrige Niederalkylgruppe bedeutet und $R^3$ eine Niederalkylgruppe bedeutet, oder ein Alkylester der Formel

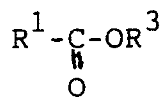

ist, worin $R^3$ wie zuvor definiert ist.

5. Verfahren gemäß Anspruch 4, in welchem die sperrige Niederalkylgruppe eine t-Butylgruppe ist.

6. Verfahren gemäß irgendeinem der vorhergehenden Ansprüche, dadurch gekennzeichnet, daß es in einem Niederalkanol-Lösungsmittel in Gegenwart eines Alkalimetallsalzes des Alkanols durchgeführt wird.

7. Verfahren gemäß Anspruch 6, dadurch gekennzeichnet, daß es in Ethanol in Gegenwart von Natriumethoxid durchgeführt wird.

8. Eine Verbindung der Formel (A) gemäß Definition in Anspruch 1.